# EUROPEAN PATENT APPLICATION

(11) **EP 4 223 322 A1**
(43) Date of publication of application: **09.08.2023**
(21) Application number: 21875897.7
(22) Date of filing: 01.10.2021
(51) Int. Cl.: A61L 2/20, A61L 101/20, A61L 101/22

(54) **DECONTAMINATION APPARATUS AND DECONTAMINATION METHOD**

(30) Priority: 02.10.2020 JP 2020167522
(71) Applicant: Nitta Corporation, Osaka-shi, Osaka 556-0022 (JP)
(72) Inventor: SHIGETA, Makoto, Yamatokooriyama-shi, Nara 639-1085 (JP); IKEDA, Takuji, Yamatokooriyama-shi, Nara 639-1085 (JP)
(74) Representative: Hasegawa, Kan
(86) International application number: PCT/JP2021/036526
(87) International publication number: WO 2022/071602

(57) **Abstract**

A decontamination apparatus is configured to perform decontamination for at least one of microorganisms and viruses present inside an object to be decontaminated. A particle removal filter is attached to the inside of the object to be decontaminated. The decontamination apparatus is equipped with a vapor generator unit and a pump. The vapor generator unit is configured to generate vapor containing peracetic acid without heating and without releasing mist. The pump is configured to suck the vapor from the exhaust side of the particle removal filter and to supply the vapor to the air supplying side of the particle removal filter. This decontamination apparatus is used in a state where the decontamination apparatus is disposed outside the object to be decontaminated.

## Description

### Technical Field

The present invention relates to a decontamination apparatus and a decontamination method.

### Background Art

Japanese Patent No. 6250491 (Patent Literature 1) discloses a decontamination apparatus configured to decontaminate the inside of a biosafety cabinet. With this decontamination apparatus, decontamination of the inside of the biosafety cabinet is performed using a mist containing peracetic acid (see Patent Literature 1).

### Citation List

### Patent Literature

Patent Literature 1: JP 6250491

### Summary of Invention

### Technical Problem

A particle removal filter, such as a HEPA (High Efficiency Particulate Air) filter may be present inside an object to be decontaminated. Examples of objects to be decontaminated include safety cabinets, incubators, isolators, and automated incubating equipment. In this case, decontamination of a particle removal filter is achieved through decontamination of the inside of the object to be decontaminated. When a particle removal filter is decontaminated using mist containing peracetic acid as with the decontamination apparatus disclosed in Patent Literature 1, the mist will quickly turn into vapor when the relative humidity in the decontamination environment is low. However, when the relative humidity exceeds 80% in particular, the evaporation rate of the mist will fall, resulting in incomplete evaporation of the mist and some mist floating in mist form. As a result, mist will collect at the particle removal filter. If droplets adhere to the particle removal filter and the particle removal filter becomes wet, this may result in increased pressure loss.

In view of this problem, the present inventor(s) considered performing decontamination of the particle removal filter with vapor containing peracetic acid in place of a mist containing peracetic acid. However, since the specific gravity of peracetic acid is high, vapor supplied to the inside of an object to be decontaminated will tend to accumulate at the bottom inside the object to be decontaminated. On the other hand, in a safety cabinet for example, the particle removal filter is often disposed near the ceiling. When this is the case, even if vapor containing peracetic acid is supplied to the inside of the object to be decontaminated, the vapor will not pass through the particle removal filter, which can result in a situation where the particle removal filter is insufficiently decontaminated.

The present invention was conceived to solve the problem described above and has an object of providing a decontamination apparatus and a decontamination method capable of suppressing the occurrence of a situation where a particle removal filter is not decontaminated even though decontamination has been performed.

### Solution to Problem

A decontamination apparatus according to an aspect of the present invention is configured to perform decontamination for at least one of microorganisms and viruses present inside an object to be decontaminated. A particle removal filter is attached to the inside of the object to be decontaminated. The decontamination apparatus is equipped with a vapor generator unit and a pump. The vapor generator unit is configured to generate, without heating and without releasing mist, vapor containing peracetic acid. The pump is configured to suck the vapor from the exhaust side of the particle removal filter and to supply the vapor to the air supplying side of the particle removal filter. This decontamination apparatus is used in a state where the decontamination apparatus is disposed outside the object to be decontaminated.

With this decontamination apparatus, vapor containing peracetic acid is sucked from the exhaust side of the particle removal filter and the vapor is also supplied to the air supplying side of the particle removal filter. This means that according to this decontamination apparatus, vapor containing peracetic acid definitely passes through the particle removal filter, which makes it possible to suppress the occurrence of situations where the particle removal filter is not decontaminated in spite of decontamination having been performed.

Since vapor is generated during decontamination, the humidity inside the object to be decontaminated will rise. If the temperature inside the object to be decontaminated were to rise during decontamination, the temperature difference between the inside and the outside of the object to be decontaminated would become large and condensation may occur inside the object to be decontaminated. During decontamination, the temperature of the pump included in the decontamination apparatus rises. Accordingly, if the decontamination apparatus (pump) were disposed inside the object to be decontaminated, the temperature inside the object to be decontaminated would rise, and condensation may occur inside the object to be decontaminated. The decontamination apparatus according to the present invention is used in a state of being disposed outside the object to be decontaminated. This means that according to this decontamination apparatus, since the temperature of the pump has almost no influence on the temperature inside the object to be decontaminated, it is possible to reduce the risk of condensation occurring inside the object to be decontaminated.

In the decontamination apparatus described above, an amount of air sucked by the pump from the exhaust side of the particle removal filter may be larger than an amount of air supplied by the pump to the air supplying side of the particle removal filter.

By doing so, the inside of the object to be decontaminated is placed in a negative pressure state. Therefore, according to this decontamination apparatus, it is possible to make it difficult for the vapor supplied to the inside of the object to be decontaminated to leak to the outside.

A decontamination method according to another aspect of the present invention is a method that performs decontamination for at least one of microorganisms and viruses using the decontamination apparatus described above. This decontamination method includes: placing a BI (Biological Indicator) on the exhaust side of the particle removal filter; and confirming a decontamination effect based on a death state of the BI after the decontamination.

According to this decontamination method, it is possible to confirm a decontamination effect for a particle removal filter based on the death state of the BI.

A decontamination method according to another aspect of the present invention is a method that performs decontamination for at least one of microorganisms and viruses using the decontamination apparatus described above. There are cases where a gap that passes through between an inside and an outside of the object to be decontaminated is formed in the object to be decontaminated. This decontamination method includes: masking the gap when the gap is formed in the object to be decontaminated; and circulating the vapor by sucking the vapor from the exhaust side of the particle removal filter and also supplying the vapor to the air supplying side of the particle removal filter.

According to this decontamination method, since the gap in the object to be decontaminated is masked, it is possible to place the inside of the object to be decontaminated in a negative pressure state during decontamination. As a result, according to this decontamination method, it is possible to make it difficult for the vapor supplied to the inside of the object to be decontaminated to leak to the outside of the object to be decontaminated.

### Advantageous Effects of Invention

According to the present invention, it is possible to provide a decontamination apparatus and a decontamination method capable of suppressing the occurrence of a situation where a particle removal filter is not decontaminated even though decontamination has been performed.

### Brief Description of Drawings

FIG. 1 depicts the overall configuration of a decontamination system;
FIG. 2 depicts the overall configuration of a decontamination apparatus;
FIG. 3 depicts a modification of the decontamination system;
FIG. 4 depicts one example of a tank including water;
FIG. 5 is a flowchart depicting one example of a decontamination procedure for an inside of a safety cabinet;
FIG. 6 depicts a modification of the decontamination apparatus;
FIG. 7 depicts a modification of a vapor generator unit; and
FIG. 8 is a graph collectively showing values of the thermo-hygrometer during decontamination.

### Description of Embodiments

Embodiments of the present invention will now be described in detail with reference to the accompanying drawings. Note that identical or corresponding parts in the drawings have been assigned the same reference numerals and description thereof is not repeated.

### 1. Configuration of Decontamination System

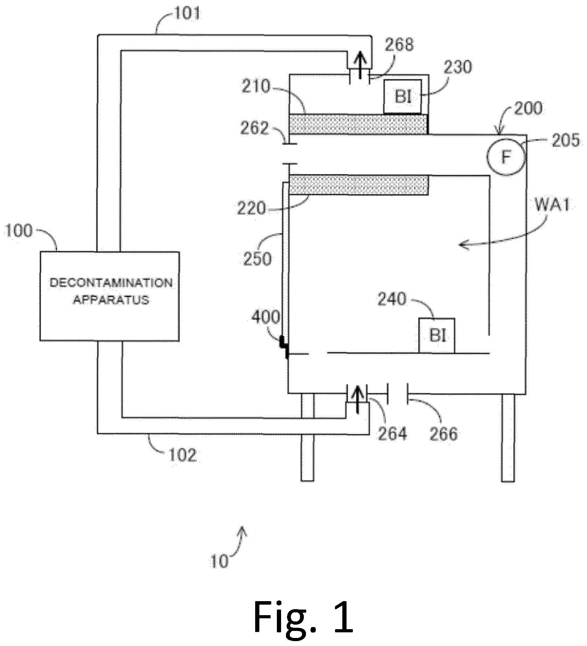

FIG. 1 depicts the overall configuration of a decontamination system 10 including a decontamination apparatus 100 according to the present embodiment. As depicted in FIG. 1, the decontamination system 10 includes the decontamination apparatus 100 and a safety cabinet 200. In the decontamination system 10, the decontamination apparatus 100 is connected to the safety cabinet 200 via pipes 101 and 102. As one example, the decontamination apparatus 100 performs decontamination to remove at least one of microorganisms and viruses present inside the safety cabinet 200. Note that in the decontamination system 10, the decontamination apparatus 100 is disposed outside the safety cabinet 200, which is the object to be decontaminated.

The safety cabinet 200 is box-shaped experimentation equipment which is used to suppress biohazards. A technician inserts his/her hands into a work area WA1 and conducts an experiment using biological materials for example. The safety cabinet 200 includes a fan 205, HEPA (High Efficiency Particulate Air) filters 210 and 220, and a shutter 250. During experiments, the fan 205 operates to generate an air flow, so that cleaned air is discharged to the outside through the HEPA filter 210 and cleaned air is supplied to the work area WA1 through the HEPA filter 220. The shutter 250 is configured to be openable and closeable.

As examples, communication holes 262, 264, 266, and 268 are formed in the safety cabinet 200. These communication holes may be provided exclusively for decontamination purposes, and as one example, a drain portion, a vacuum, and a DOP sampling port provided on a typical safety cabinet may be used as the communication holes. Each of the communication holes 262, 264, 266, and 268 is openable and closeable. In the example in FIG. 1, the decontamination apparatus 100 is connected to the communication hole 264 via the pipe 102 and the decontamination apparatus 100 is connected to the communication hole 268 via the pipe 101. That is, the decontamination apparatus 100 is connected to the communication hole 268 in a ceiling portion of the safety cabinet 200 and to the communication hole 264 in a bottom portion of the safety cabinet 200. The pipe 102 may be connected to one or two or more communication holes.

During decontamination, the shutter 250 is closed. However, even when the shutter 250 is closed, the inside and the outside of the safety cabinet 200 may not be completely shut off, and in such case, a slight gap may be formed. During decontamination, depending on the extent to which leakage of the decontamination gas occurs, the gap may be covered with masking tape 400 to prevent leakage of the decontamination gas. It is also preferable for the communication holes 262 and 266 to be closable.

Prior to decontamination, a BI (Biological Indicator) 230 is placed in the space above the HEPA filter 210. A BI 240 is similarly placed inside the work area WA1. After decontamination has been completed, the BIs 230 and 240 are cultured and the effect of decontamination is confirmed based on a death state for the BIs 230 and 240.

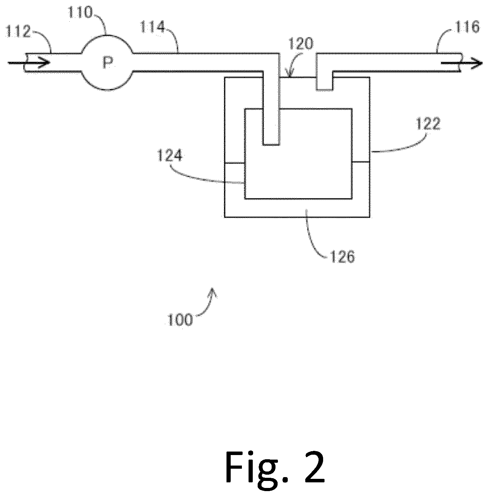

FIG. 2 depicts the overall configuration of the decontamination apparatus 100. As depicted in FIG. 2, the decontamination apparatus 100 includes a pump 110 and a vapor generator unit 120. Pipes 112 and 114 are connected to the pump 110. The pipe 112 is connected to the pipe 101. The pump 110 is configured to suck air from the pipe 112 side and to supply air to the pipe 114 side.

The vapor generator unit 120 is configured to generate only vapor containing peracetic acid, without heating a chemical solution 126 and without releasing mist. The vapor generator unit 120 includes a container 122, a moisture absorbing member 124, and the chemical solution 126. As one example, the container 122 is a sealed container that is cylindrical. The container 122 holds the moisture absorbing member 124 and the chemical solution 126. The chemical solution 126 is a medical agent in liquid form containing peracetic acid. That is, the chemical solution 126 is a peracetic acid preparation. The moisture absorbing member 124 is made of a porous material, for example. The moisture absorbing member 124 is immersed in the chemical solution 126. The moisture absorbing member 124 sucks up the chemical solution 126 inside the container 122 by capillary action. That is, the moisture absorbing member 124 becomes soaked with the chemical solution 126. There are no particular limitations on the positions and lengths inside the sealed container (that is, the container 122) of the connecting pipes (that is, the pipes 114 and 116) that connect to the sealed container. However, since it is preferable for connecting pipes to not enter the chemical solution 126 and cause foaming of the chemical solution 126, it is preferable for the positions and lengths of the connecting pipes inside the sealed container to be set to achieve this (that is, for the tips of the connecting pipes to not enter the chemical solution 126) . It is also preferable for the tips of the pipes 114 and 116 to be positioned so that the tip of the connecting pipe for introducing air (that is, the pipe 114) and the tip of the connecting pipe for exhausting air (that is, the pipe 116) are physically separated, so that the air flow path is long and generation of peracetic acid vapor at the moisture absorbing member 124 is promoted.

Note that so long as the moisture absorbing member 124 becomes moistened with the chemical solution 126 and the chemical solution 126 can be efficiently gasified (vaporized) by passing air, there are no particular limitations on the structure and/or material of the moisture absorbing member 124. As one example, the moisture absorbing member 124 may be formed of a sheet-like material, such as woven fabric, knitted fabric, non-woven fabric, or a film, which may be used in that state or may be formed into a pleated or corrugated shape. A porous material, such as silica gel or zeolite, may be encapsulated in woven fabric, knitted fabric, non-woven fabric, a film, or the like.

According to the decontamination apparatus 100, since the chemical solution 126 is not heated, it is possible to suppress decomposition of the peracetic acid and efficiently generate peracetic acid vapor. Additionally, according to the decontamination apparatus 100, the temperature of the chemical solution 126 containing peracetic acid is kept at a similar temperature to the temperature of the space in which the HEPA filters 210 and 220 to be decontaminated are placed, which makes it possible to suppress the risk of condensation occurring within the space.

The moisture absorbing member 124 is contacted by air supplied from the pump 110 via the pipe 114, which promotes vaporization of the chemical solution 126 that has soaked into the moisture absorbing member 124. As a result, vapor containing peracetic acid (hereinafter, also referred to as "peracetic acid vapor") is generated. The peracetic acid vapor is supplied to the inside of the safety cabinet 200 via the pipe 116. Note that the pipe 116 is connected to the pipe 102.

In this way, in the decontamination system 10, the inside of the safety cabinet 200 is decontaminated by the peracetic acid vapor. Through decontamination of the inside of the safety cabinet 200, the HEPA filters 210 and 220 are also decontaminated. The reason why the decontamination system 10 performs decontamination using peracetic acid vapor instead of mist containing peracetic acid is described below. In other words, the reason why vaporization (vapor) is used and not atomization (mist) will be described.

When decontamination of the HEPA filters 210 and 220 is performed with a mist containing peracetic acid, mist will collect at the HEPA filters 210 and 220. As a result, at the HEPA filters 210 and 220, there is the risk of increased pressure loss due to wetting by the collected mist and/or accelerated deterioration due to the decontaminating agent.

The peracetic acid vapor generated by vaporization does not collect as particles at the HEPA filters 210 and 220. Accordingly, the above problems with atomization do not occur. Vaporization is adopted in the decontamination system 10 for this reason.

The pump 110 is configured to suck air containing peracetic acid vapor from the exhaust side of the HEPA filter 210 and to supply air containing peracetic acid vapor to the air supplying side of the HEPA filter 210. Note that the amount of air sucked by the pump 110 from the exhaust side of the HEPA filter 210 is larger than the amount of air supplied by the pump 110 to the air supplying side of the HEPA filter 210. This can be achieved, for example, by allowing some air in the pipe 102 to leak. Since the leaked air contains decontamination gas, it is preferable to discharge air to the outside after absorbing the decontamination gas with a chemical filter such as activated carbon. Aside from the pipe 102, leaks may be provided at other locations, like the pump on the pipe 114 and the pipe 116.

Since the amount of air sucked by the pump 110 is larger than the amount of air supplied, the inside of the safety cabinet 200 is placed in a negative pressure state during decontamination. By doing so, it is possible to prevent the peracetic acid vapor from leaking to the outside of the safety cabinet 200.

When the inside of the safety cabinet 200 is placed in a negative pressure state, it is preferable to prevent the negative pressure from becoming excessively high. If the negative pressure becomes too high, there is the risk of denting of the side walls of the safety cabinet 200 and other problems.

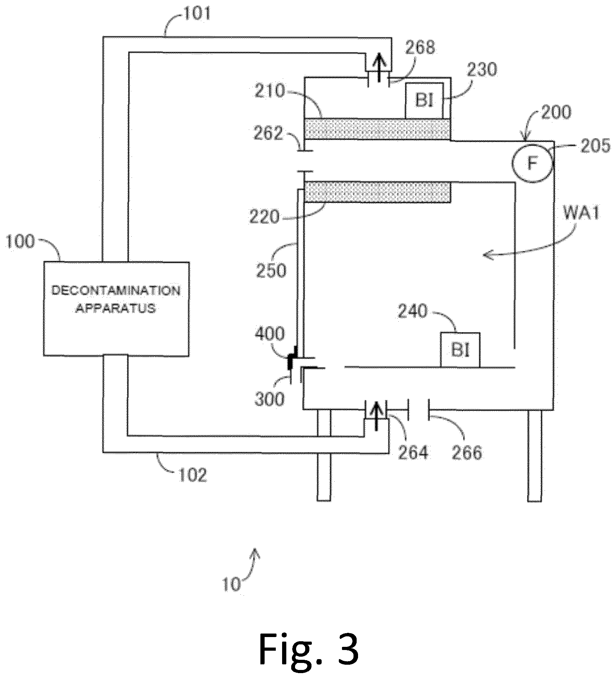

To avoid excessive depressurization, it is preferable to provide an opening in the safety cabinet 200. As examples, communication holes such as the communication holes 262 and 266 may be opened, and as depicted in FIG. 3, a gap may be provided in a lower part of the shutter 250, and a tube 300 may be placed in this gap of the shutter 250. Such openings pass through between the inside and outside of the safety cabinet 200 even during decontamination. Note that the inner diameters of the tube 300 and the communication holes are 1 mm to 3 cm, for example. In addition, during decontamination, the gap at the lower part of the shutter 250 aside from the tube 300 is covered by the masking tape 400.

Note that with the decontamination system 10, since openings are provided in the safety cabinet 200 during decontamination and air is introduced into the inside of the safety cabinet 200 through these openings, the inside of the safety cabinet 200 does not become excessively depressurized.

Also, to achieve a sufficient decontamination effect, it is necessary to maintain a certain level of humidity inside the safety cabinet 200. On the other hand, when the humidity inside the safety cabinet 200 becomes excessively high, condensation will occur inside the safety cabinet 200. Condensation causes corrosion of internal parts of the safety cabinet 200. With the decontamination system 10, air is introduced into the safety cabinet 200 through the tube 300 and the opened communication holes (or "openings") 262 and 266, which prevents the humidity inside the safety cabinet 200 from becoming higher than necessary.

As one example, parts of the pipes 114, 116, and 102 on the exhaust side of the pump 110 can be provided with closable openings for discharging the air that circulates inside the safety cabinet 200, the decontamination apparatus 100, and the pipes 101 and 102. If the humidity inside the safety cabinet 200 has become too high, it is possible to discharge the air circulating inside the safety cabinet 200 from the openings and to increase the amount of air introduced into the safety cabinet 200 via the tube 300 and the opened communication holes (openings) 262 and 266 to lower the humidity inside the safety cabinet 200. It is preferable for the air that is released to be discharged after the decontamination gas has been recovered by a chemical filter or the like.

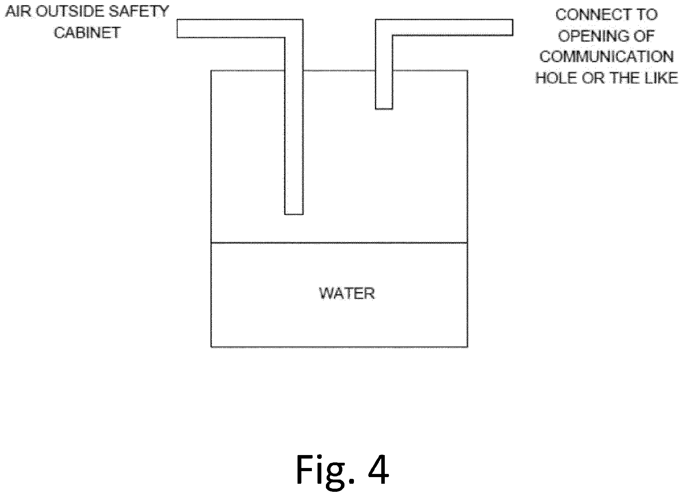

As one example, if a tank containing water like that depicted in FIG. 4 is placed at the tube 300 or the opened communication holes 262 and 266, high-humidity air will be introduced into the safety cabinet 200 via the tube 300 or the opened communication holes 262 and 266, which makes it possible to raise the humidity inside the safety cabinet 200. This is effective when the humidity of the air outside the safety cabinet 200 is low and the humidity inside the safety cabinet 200 has become excessively low. That is, the humidity inside the safety cabinet 200 can be controlled by using the tube 300 or the openings of the opened communication holes 262 and 266. Note that as the amount of air introduced into the safety cabinet 200 through the tube 300 or the openings of the opened communication holes 262 and 266 increases, the concentration of the peracetic acid vapor will decrease. Accordingly, the amount of air introduced into the safety cabinet 200 through the tube 300 or the opened communication holes 262 and 266 should preferably be 3% or less, for example, of the amount of air circulating in the decontamination system 10.

It is also preferable for the fan 205 to be stopped during decontamination. This is because when the fan 205 operates and emits heat, the temperature of the air inside the safety cabinet 200 will rise, which can cause condensation.

### 2. Decontamination Procedure

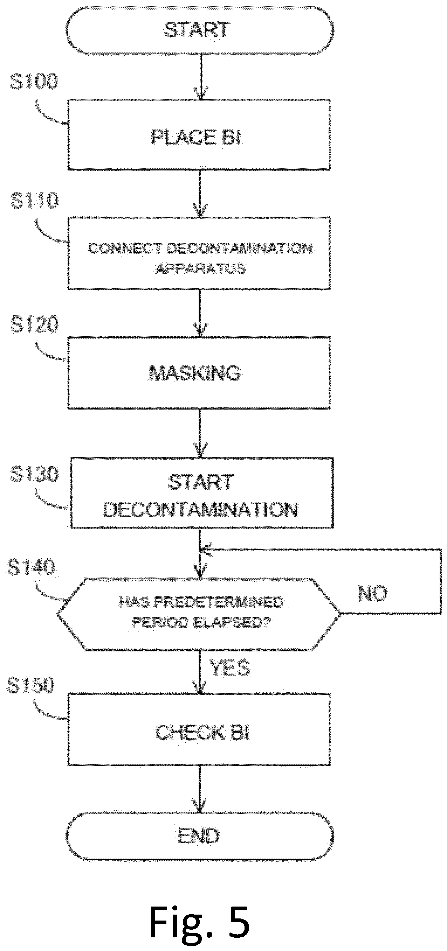

FIG. 5 is a flowchart depicting an example decontamination procedure for the inside of the safety cabinet 200. Each step depicted in this flowchart is performed by an operator.

As depicted in FIG. 5, the operator places the BIs 230 and 240 inside the safety cabinet 200 (step S100). The operator connects the decontamination apparatus 100 to the safety cabinet 200 using the pipes 101 and 102 (step S110). As necessary, the operator provides openings in the safety cabinet 200 and covers gaps with the masking tape 400 (step S120). The operator operates the decontamination apparatus 100 to start decontamination of the inside of the safety cabinet 200 (step S130).

The operator determines whether a predetermined period has elapsed (step S140). The operator stands by until the predetermined period elapses (NO in step S140). When the predetermined period has elapsed (YES in step S140), the operator confirms the decontamination effect based on a death state for the BIs 230 and 240 (step S150). Decontamination of the inside of the safety cabinet 200 is completed by confirming a death state for the BIs 230 and 240.

### 3. Features

As described above, with the decontamination apparatus 100, vapor containing peracetic acid is sucked from the exhaust side of the HEPA filter 210 and the vapor is supplied to the air supplying side of the HEPA filter 210. If sucking of the peracetic acid vapor were not performed, since the specific gravity of the peracetic acid is high, the peracetic acid vapor supplied to the inside of the safety cabinet 200 would tend to accumulate at the bottom portion inside the safety cabinet 200. Were the peracetic acid vapor to accumulate at the bottom portion of the safety cabinet 200, the peracetic acid vapor would not pass through the HEPA filter 210, which may result in a situation where the HEPA filter 210 is not sufficiently decontaminated.

As described earlier, with the decontamination apparatus 100, peracetic acid vapor is sucked from the exhaust side of the HEPA filter 210 and peracetic acid vapor is also supplied to the air supplying side of the HEPA filter 210. Accordingly, by using the decontamination apparatus 100, the peracetic acid vapor will definitely pass through the HEPA filter 210, which makes it possible to suppress the occurrence of situations where the HEPA filter 210 is not decontaminated in spite of decontamination having been performed.

Also, during decontamination, since an aqueous solution containing peracetic acid is vaporized, the humidity inside the safety cabinet 200 will rise. If the temperature inside the safety cabinet 200 were to rise during decontamination, the temperature difference between the inside and the outside of the safety cabinet 200 would increase so that condensation may occur inside the safety cabinet 200. During decontamination, the temperature of the pump 110 included in the decontamination apparatus 100 rises. Accordingly, if the pump 110 were disposed inside the safety cabinet 200, the temperature inside the safety cabinet 200 would rise and condensation may occur inside the safety cabinet 200. On the other hand, the decontamination apparatus 100 is used in a state where the decontamination apparatus 100 is disposed outside the safety cabinet 200. Accordingly, with the decontamination apparatus 100, since the temperature of the pump 110 has almost no influence on the temperature inside the safety cabinet 200, it is possible to reduce the risk of condensation occurring inside the safety cabinet 200.

### 4. Modifications

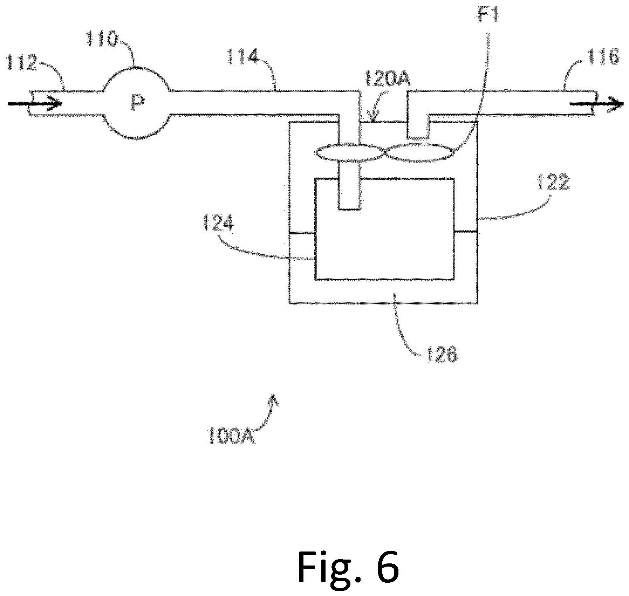

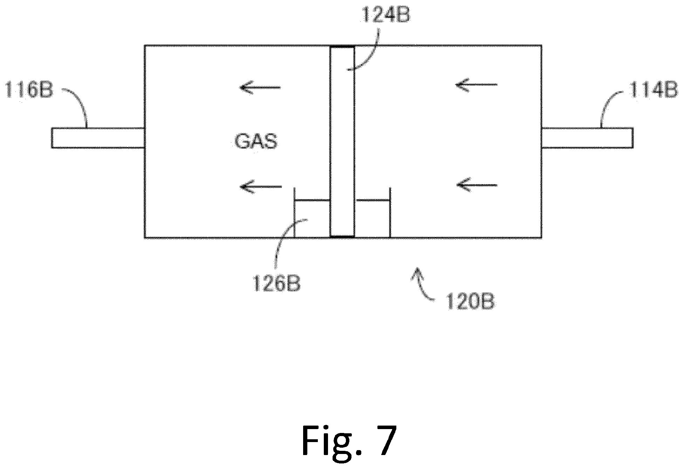

Although an embodiment has been described above, the present invention is not limited to the above embodiment and various modifications can be made without departing from the spirit of the present invention. Modifications are described below.
4-1
   In the embodiment described above, the particle removal filters disposed in the safety cabinet 200 are HEPA filters 210 and 220. However, the particle removal filters disposed inside the safety cabinet 200 are not limited to this. As examples, the particle removal filters to be decontaminated may be medium performance filters or ULPA filters.
4-2
   In the above embodiment, the object to be decontaminated is the safety cabinet 200. However, the object to be decontaminated is not limited to this. As one example, the object to be decontaminated may be any container so long as the container can internally accommodate a particle removal filter. The object to be decontaminated may be hermetically sealed or may be in a semi-sealed state. The expression "semi-sealed state" refers to a state which is close to being sealed but is not completely sealed. As one example, this is a state where air is blocked to a certain extent between the inside and the outside of the safety cabinet 200 so that there is no extreme drop in the concentration of the peracetic acid vapor due to the leakage of the peracetic acid vapor. As examples, the object to be decontaminated may be an isolator apparatus, an incubator, a centrifuge, a pass box, a storage cabinet, an air conditioner, a clean bench, a duct, or the like.
4-3
   In the decontamination apparatus 100 depicted in FIG. 2, air flows between the safety cabinet 200, the pump 110, the vapor generator unit 120, and the safety cabinet 200 in that order, but the order in which air flows is not limited to this. As one example, air may flow between the safety cabinet 200, the vapor generator unit 120, the pump 110, and the safety cabinet 200 in that order.
4-4
   The structure of the vapor generator unit 120 may be any structure that generates peracetic acid vapor without generating mist. As one example, as depicted in FIG. 6, a stirring fan F1 for promoting vaporization may be installed in a vapor generator unit 120A, and if the vaporization is sufficient, the porous body 124 may be omitted. Also, the construction of the vapor generator unit 120 may be as depicted in FIG. 7. That is, in a vapor generator unit 120B, a moisture absorbing member 124B sucks up a chemical solution 126B by capillary action. Peracetic acid vapor (gas) flows toward a pipe 116B due to air introduced into the vapor generator unit 120B through a pipe 114B. The peracetic acid vapor is introduced into the safety cabinet 200 via the pipe 116B.
4-5
   It is sufficient for the pump 110 to be capable of sucking and exhausting air with sufficient performance to circulate air in keeping with the present invention. Examples include a centrifugal blower, an axial blower, a mixed flow blower, a cross flow blower, a diaphragm pump, a piston pump, and a plunger pump. However, there are no particular limitations on the structure and size of the pump 110.

### 5. Experiment

The following experiment was conducted to confirm the effect of the present invention. The content of the experiment and the experiment results are described below.

In the experiment, the decontamination system 10 depicted in FIG. 1 was prepared. As the safety cabinet 200, an "MHE-181AB3" manufactured by PHC Corporation was used and installed in a location that was not directly contacted by air expelled by an air conditioner in the room. As the chemical solution 126 (see FIG. 2), "Mincare" manufactured by Cantel Medical Corp. was used. "HMV-091" with a bacterial count of 106 manufactured by MesaLab was used as the BIs 230 and 240. As the culture medium for the BIs 230 and 240, "PM/100" manufactured by MesaLab was used. As a thermo-hygrometer for measuring the temperature and humidity inside the safety cabinet 200, a "LR5001" temperature-humidity logger manufactured by Hioki Co., Ltd. was used.

The decontamination apparatus 100 and the safety cabinet 200 were connected using the pipes 101 and 102 so as to suck air from the downstream side of the HEPA filter 210 and to supply air into the safety cabinet 200 from a drain portion. Masking of the safety cabinet 200 was performed to close the communication holes 262 and 266 and place the inside of the safety cabinet 200 in a semi-sealed state. The tube 300 was attached to a gap in the safety cabinet 200. The pump 110 was operated with a pressure gauge attached to the tube 300. The needle of the pressure gauge advanced toward negative pressure. A flow meter was also attached to the tube 300 to measure the flow rate. The flow rate was 5 ml/min.

Mincare (with a peracetic acid content of 4.5%) diluted with pure water was used as the chemical solution 126. The dilution ratio was 10%. As the container 122, a cylindrical sealed container with an inner diameter of around 200 mm and a height of around 300 mm was used. The container 122 was filled with 1 L of the chemical solution 126. A cylindrical moisture absorbing member 124 was disposed around the inner circumference of the container 122.

The BIs 230 and 240 were respectively placed above the HEPA filter 210 and inside the work area WA1. After decontamination, the BIs 230 and 240 were cultured.

The circulating flow rate of the pump 110 was 120 L/min. The decontamination period was 185 minutes. The humidity inside the safety cabinet 200 after 3 hours was RH94%.

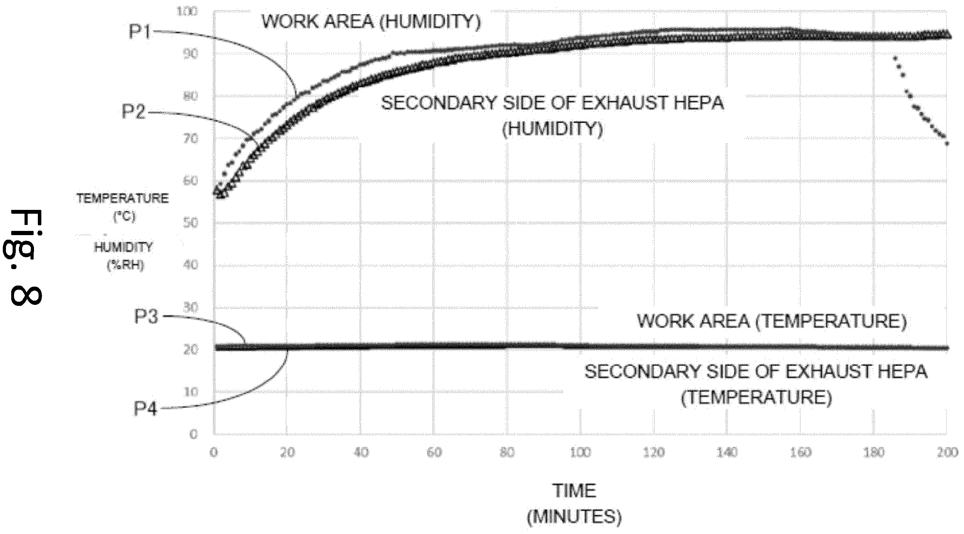

FIG. 8 is a graph collectively showing values of the thermo-hygrometer during decontamination. In FIG. 8, each point P1 indicates the humidity in the work area WA1 and each point P2 indicates the humidity above the HEPA filter 210. Each point P3 indicates the temperature in the work area WA1, and each point P4 indicates the temperature above the HEPA filter 210. In this experiment, condensation did not occur inside the safety cabinet 200.

After decontamination was completed, the BIs 230 and 240 were cultured. The BIs 230 and 240 were completely dead (negative). Also, since the pump 110 was disposed outside the safety cabinet 200, no rise in temperature was observed inside the safety cabinet 200.

### List of Reference Numerals

10 Decontamination system
100 Decontamination apparatus
101, 102, 112, 114, 116 Pipe
110 Pump
120 Vapor generator unit
122 Container
124 Moisture absorbing member
126 Chemical solution
200 Safety cabinet
205 Fan
210, 220 HEPA filter
230, 240 BI
250 Shutter
262, 264, 266, 268 Communication hole
300 Tube
400 Masking tape
P1, P2, P3, P4 Point
WA1 Work area

## Claims

1. A decontamination apparatus configured to decontaminate at least one of microorganisms and viruses present inside an object to be decontaminated, the object to be decontaminated having a particle removal filter attached to an inside thereof and the decontamination apparatus comprising:
a vapor generator unit configured to generate, without heating and without releasing mist, vapor containing peracetic acid; and
a pump configured to suck the vapor from an exhaust side of the particle removal filter and to supply the vapor to the air supplying side of the particle removal filter,
wherein the decontamination apparatus is used in a state of being disposed outside the object to be decontaminated.

2. The decontamination apparatus according to claim 1,
wherein an amount of air sucked by the pump from the exhaust side of the particle removal filter is larger than an amount of air supplied by the pump to the air supplying side of the particle removal filter.

3. A decontamination method of performing decontamination for at least one of microorganisms and viruses using the decontamination apparatus according to claim 1 or 2, comprising:
placing a BI (Biological Indicator) on the exhaust side of the particle removal filter; and
confirming a decontamination effect based on a death state of the BI after the decontamination.

4. A decontamination method of performing decontamination for at least one of microorganisms and viruses using the decontamination apparatus according to claim 1 or 2,
wherein a gap that connects an inside and an outside of the object to be decontaminated is formed in the object to be decontaminated, and
the decontamination method comprises:
masking the gap; and
circulating the vapor by sucking the vapor from the exhaust side of the particle removal filter and supplying the vapor to the air supplying side of the particle removal filter.
